# EUROPÄISCHE PATENTSCHRIFT

(11) **EP 2 926 771 B1**
(45) Veröffentlichungstag und Bekanntmachung des Hinweises auf die Patenterteilung: **21.06.2017**
(21) Anmeldenummer: 15155159.5
(22) Anmeldetag: 16.02.2015
(51) Int. Cl.: A61F 2/18, H04R 25/00

(54) **Gehörknöchelchenprothese mit längs-perforierter Schlinge**
Auditory ossicle prosthesis with loop with longitudinal perforation
Prothèse d'osselet dotée d'une boucle perforée dans le sens de la longueur

(30) Priorität: 31.03.2014 DE 202014101511 U
(43) Veröffentlichungstag der Anmeldung: 07.10.2015
(73) Patentinhaber: Heinz Kurz GmbH Medizintechnik, 72144 Dusslingen (DE)
(72) Erfinder: Kartush, Jack M., Bloomfield Hills, 48302-2323 (US); Steinhardt, Uwe, 72145 Hirrlingen (DE); Kurz, Heinz, 72144 Dusslingen (DE)
(74) Vertreter: Kohler Schmid Möbus Patentanwälte

(56) Entgegenhaltungen:
- EP-A1- 1 759 662
- DE-U1-202014 100 093
- US-A- 5 514 177

## Beschreibung

Die Erfindung betrifft eine passive Gehörknöchelchenprothese, die zum Ersatz oder Überbrücken mindestens eines Elements der menschlichen Gehörknöchelchenkette ausgebildet ist, mit einem länglichen, Schall übertragenden Prothesenkörper, welcher an seinem einen Ende ein erstes Ankoppelelement aufweist, das zur mechanischen Verbindung mit dem Ambossfortsatz (="Incus"), mit dem Hammergriff ("Malleus") oder mit einem länglichen Aktor-Endstück eines aktiven Hörgeräts in Form einer das Glied der Gehörknöchelchenkette oder das Aktor-Endstück umgebenden, durch eine spaltartige Öffnung nach außen hin teilweise offenen Schlinge aus bandförmigem metallischem Material gestaltet ist, welche postoperativ den Ambossfortsatz, den Hammergriff oder das Aktor-Endstück kraftschlüssig umgreift und intraoperativ im Mittelohr zur Fixierung und dauerhaften Befestigung des ersten Ankoppelelements am Ambossfortsatz, am Hammergriff oder am Aktor-Endstück mittels einer Crimpzange verformt werden kann, wobei der Prothesenkörper an seinem anderen Ende ein zweites Ankoppelelement zur mechanischen Verbindung mit einem weiteren Glied oder Teilen eines Gliedes der Gehörknöchelchenkette oder direkt mit dem Innenohr aufweist.

Eine solche passive Gehörknöchelchenprothese ist dargestellt und gezeigt in DE 20 2014 100 093 U1.

Dort ist insbesondere auch die spezielle Problematik der intra-operativen Vercrimpung des schlingenförmigen Teils des ersten Ankoppelelements ausführlich beschrieben, allerdings lediglich in Zusammenhang mit der Überbrückung von Teilen oder Gliedern der Gehörknöchelchenkette, jedoch nicht zur Ankopplung der passiven Prothese an ein Aktor-Endstück eines aktiven Hörgerätes.

Ähnliche, in der Regel als Stapes-Prothesen ausgestaltete passive Gehörknöchelchenprothesen sind schon lange bekannt beispielsweise aus US-A 3,711,869 oder US-A 3,931,648, wobei dort allerdings das erste Ankoppelelement nicht als Schlinge, sondern eher als Clip ausgeführt ist, welcher aufgrund seiner Federeigenschaften selbstverschließend und -haltend sein soll und daher nicht - wie die Schlinge einer gattungsgemäßen Prothese - vercrimpt wird.

Bei den etwa aus EP 2 385 808 B1 oder aus EP 2 583 639 A1 bekannten Gehörknöchelchenprothesen hinwiederum ist zwar das erste Ankoppelelement aus bandförmigem flexiblen Material aufgebaut und mit Schlitzen parallel zur Längsachse des zu umfassenden Gliedes der Gehörknöchelchenkette versehen. Allerdings ist der geometrische Aufbau des dort verwendeten Ankoppelelements nicht schlingenförmig, sondern als Schlaufe ausgeführt, deren freies Ende intra-operativ in eine dafür vorgesehene Öse am Prothesenkörper eingesteckt wird. Ein dem Vercrimpungsvorgang beim intra-operativen Fixieren der Schlinge gemäß der eingangs zitierten DE 20 2014 100 093 U1 vergleichbarer Befestigungsschritt erfolgt bei diesen bekannten Prothesen daher ebenfalls nicht.

Gehörknöchelchenprothesen, bei denen das erste Ankoppelelement in der Tat als Schlinge ausgeführt ist, sind etwa in US 6,554,861 B1 oder in DE 10 2007 851 B3 gezeigt und beschrieben. Jedoch ist dort - im Gegensatz zu den eingangs zitierten gattungsgemäßen Prothesen - die Schlinge nicht aus bandförmigem metallischem Material hergestellt, sondern aus Runddraht-Material, so dass sich der Vercrimpungsvorgang beim intra-operativen Befestigen der Schlinge wiederum erheblich anders als bei den gattungsgemäßen Prothesen gestaltet.

Das menschliche Mittelohr mit seinen Gehörknöchelchen hat die Funktion, die über den äußeren Gehörgang auf das Trommelfell auftreffenden Schallwellen auf das mit Flüssigkeit gefüllte Innenohr zu übertragen. Die drei Gehörknöchelchen sind der am Trommelfell befestigte Hammer (lat. malleus), der Steigbügel (lat. stapes), der über seine Fußplatte (lat. basis stapedis) mit dem Innenohr verbunden ist, und der Amboss (lat. incus), der sich zwischen dem Hammer und dem Steigbügel befindet und mit diesen gelenkig verbunden ist. Beispielsweise die Otosklerose ist eine Erkrankung des menschlichen Felsenbeins (= Knochen, in dem das gesamte Ohr sitzt), bei der es durch entzündungsähnliche Knochenumbauprozesse zu einer Fixierung des normalerweise locker schwingenden Steigbügels kommen kann. Dadurch wird das Schallsignal nicht oder nur unvollkommen über die Gehörknöchelchenkette auf das Innenohr übertragen, was zur Schwerhörigkeit führt.

Gehörknöchelchenprothesen dienen allgemein der Verbesserung der Schallübertragung bei unterschiedlichen pathologischen Befunden. Sie werden verwendet, um bei ganz oder teilweise fehlenden oder geschädigten Gehörknöchelchen des menschlichen Mittelohrs die Schallübertragung vom Trommelfell zum Innenohr zu verbessern oder überhaupt erst zu ermöglichen. Die passive Gehörknöchelchenprothese weist dabei zwei Enden auf, wobei je nach den konkreten Gegebenheiten das eine Ende der Gehörknöchelchenprothese am Trommelfell, einem Gehörknöchelchen oder dem Aktor-Endstück eines aktiven elektronischen Hörgeräts und ihr anderes Ende beispielsweise am Steigbügel der menschlichen Gehörknöchelchenkette befestigt oder direkt ins Innenohr getaucht wird.

Drei besonders häufig verwendete Arten von Gehörknöchelchenprothesen sind die Steigbügel-Prothesen, die Partial-Prothesen und die Total-Prothesen. Steigbügel-Prothesen (=Stapes-Prothesen) werden in der Regel am langen Ambossfortsatz fixiert und ragen über einen Stempel (=Piston) ins Innenohr oder sitzen mit dem Stempel auf einem Gewebestück auf, welches das Innenohr abdichtet. Partial-Prothesen liegen meist mit einer Kopfplatte am Trommelfell an und stellen eine Verbindung zum Steigbügelköpfchen her. Total-Prothesen verbinden das Trommelfell mit der Steigbügel-Fußplatte.

Des Weiteren unterscheidet man generell zwischen *passiven* Gehörknöchelchenprothesen und *aktiven* Hör-Implantaten mit elektronischen Verstärkerelementen, die über ein externes Energieteil betrieben werden. Bei der eingangs definierten gattungsgemäßen Gehörknöchelchenprothese handelt es sich um eine passive Prothese, bei der am oberen Ende des Prothesenkörpers ein Ankoppelelement vorgesehen ist, welches mit dem Ambossfortsatz (="Incus"), mit dem Hammergriff ("Malleus") oder einem Aktor-Endstück eines aktiven Hörgeräts vercrimpt wird.

In der wissenschaftlichen Literatur sind die Methoden und Probleme bei der Befestigung derartiger Gehörknöchelchenprothesen mit schlingenförmigem Ankoppelelement vielfältig dargestellt.

Eigens für die Verbindung mit dem Incus ausgestaltete Stapes-Prothesen, welche als erstes Ankoppelelement jeweils eine Schlinge, eine Schlaufe oder einen Clip zur Vercrimpung um den Incus aufweisen, sind in ihrem geometrischen Aufbau, bezüglich der Details ihrer Ankoppelung am Incus sowie hinsichtlich ihrer Funktionsweise ausführlich beschrieben etwa in
Fritsch et al., "History of Otology: Phylogeny of the Stapes Prosthesis", Otology & Neurotology, 29:407-415, 2008
oder in
Fontana et al., "A scanning electron microscopic study of crimping of stapedial prostheses", Auris Nasus Larynx 39 (2012) 461-468
oder in
Kwok et al., "Stapes Surgery: How Precisely Do Different Prostheses Attach to the Long Process of Incus with Different Instruments and Different Surgeons?", Otology & Neurotology, 23:289-295, 2002
oder in dem Lehrbuch "Middle Ear Surgery" von Hildmann und Sudhoff, erschienen im Springer-Verlag Berlin Heidelberg, ISBN: 978-3-540-22201-9, 2006, insbesondere auf Seite 117.

### Auch in dem Artikel

Huber et al., "Stapes Prosthesis Attachment: The Effect of Crimping on Sound Transfer in Otosclerosis Surgery", Laryngoscope 113: May 2003 werden die Probleme diskutiert, welche sich durch die gängige Vercrimp-Technik derartiger Stapes-Prothesen im Einzelnen ergeben können.

Die bekannten Crimp-Stapesprothesen -meist mit Piston zur Ankopplung an das Innenohr am unteren Ende der Prothese- werden in der Regel mittels eines Crimp- oder eines Hechtmaulzängchens beispielsweise am langen Ambossfortsatz appliziert. Diese Applikation besteht zum einem aus dem einfachen Aufsetzen oder Auflegen der Schlinge auf den Amboss. Der nächste Schritt besteht darin, die Schlinge mit dem Zängchen zu schließen. Dieser Schließvorgang gilt auch heute noch als der schwierigste Teil der Mittelohr-Operation und zwar deshalb, da aufgrund des Schließvorgangs, der Piston ins Innenohr nach medial bewegt werden kann. Diese Bewegungen können zu kleineren, aber auch gravierenden Innenohrschädigungen führen, welche auf den post-operativen Gesundheitszustand des Patienten negativ nachwirkend sein können.

Da der Amboss aufgrund anatomischer Variation quasi nie rund oder oval ist, sondern in der Regel einen sehr komplexen Querschnitt aufweist, ist es von Vorteil, wenn die Schlinge eine möglichst geringe Auffederung ("Memory Spring Effect") hat. Wird also die Schlinge mit dem Zängchen geschlossen, möchte der Chirurg eine möglichst sanfte, weiche und dauerhaft bleibende plastische Verformung ohne nachfolgende Rückfederung bewirken.

Nachteilig bei den eingangs beschriebenen, etwa aus DE 20 2014 100 093 U1 bekannten gattungsgemäßen Gehörknöchelchenprothesen ist daher einerseits die stets vorhandene Neigung zum Zurückfedern des flachen metallischen Materials der Schlinge während des Vercrimpens aufgrund des oben erwähnten mechanischen Auffederungseffekts, welcher sich beim Verbiegen des Bandmaterials bemerkbar macht. Andererseits verursacht auch die intrinsische Steifigkeit des Materials Probleme im Hinblick auf ein möglichst "zielgenaues" und dauerhaftes Verformen des Ankoppelelements bei der intra-operativen Verarbeitung.

Die Patentschrift US 5,514,177 offenbart eine Gehörknöchelchenprothese gemäß dem Oberbegriff von Anspruch 1

Aufgabe der vorliegenden Erfindung ist es demgegenüber, eine gattungsgemäße passive Gehörknöchelchenprothese der eingangs beschriebenen Art mit möglichst simplen technischen Mitteln und wirtschaftlich preisgünstig dahin gehend zu verbessern, dass sie einerseits fertigungstechnisch besonders einfach und preiswert herzustellen ist, und bei der andererseits die oben beschriebenen mechanischen Auffederungseffekte nicht oder deutlich vermindert auftreten, wobei auch die intrinsische Steifigkeit des Materials möglichst stark verringert sein soll.

Erfindungsgemäß wird diese relativ komplexe und anspruchsvolle Aufgabe auf ebenso überraschend einfache wie wirkungsvolle Art und Weise dadurch gelöst, dass die Schlinge aus bandförmigem metallischem Material eine Vielzahl von länglichen Perforationen aufweist, deren Längsachsen im implantierten Zustand der Gehörknöchelchenprothese jeweils auf einer gekrümmten Bahn rechtwinklig oder schräg zu einer zur Längsachse des Ambossfortsatzes, des Hammergriffs oder des Aktor-Endstücks parallelen Achse verlaufen.

In einem -gedachten- flachen Zustand der im implantierten Zustand gekrümmten Schlinge verlaufen also die länglichen Perforationen in Längsrichtung des flachen bandförmigen Materials der Schlinge oder immerhin unter einem spitzen Winkel zur Längsrichtung des Bandes, so dass sie im implantierten Zustand der Gehörknöchelchenprothese jeweils auf einer gekrümmten Bahn rechtwinklig oder schräg zu einer zur Längsachse des zu umgreifenden Gliedes der Gehörknöchelchenkette beziehungsweise des länglichen Aktor-Endstücks verlaufen.

Auf diese Weise kann einerseits die Federwirkung des flachen Bandmaterials der Schlinge wesentlich verringert werden, andererseits ist auch die Kraft, die zur Vercrimpung aufgewendet werden muss, deutlich kleiner, nämlich schätzungsweise um ca. 50%.

Im Unterschied zur Gehörknöchelchenprothese nach der oben diskutierten EP 2 385 808 B1, die Schlitzen parallel zur Längsachse des zu umfassenden Gliedes der Gehörknöchelchenkette aufweist, verlaufen die Perforationen der erfindungsgemäßen Prothese jeweils auf einer gekrümmten Bahn rechtwinklig oder schräg zu einer zur Längsachse des zu umgreifenden Teils parallelen Achse, also im Wesentlichen parallel zum Schaft der Prothese, während die Perforationen bei der Schlaufe gemäß EP 2 385 808 B1 quer dazu verlaufenden. Außerdem benötigt die erfindungsgemäße Prothese mit ihrer offenen Schlinge keine das freie Schlaufenende aufnehmende Öse oder einen anderen derartigen Mechanismus, in welchen die Schlaufe eingeführt werden muss.

Die Schlinge mit den erfindungsgemäß vorgeschriebenen Längsperforationen hat im Vergleich zur Schlaufe mit den Querperforationen nach EP 2 385 808 B1 unter anderem auch den Vorteil, dass sie nicht an der schwachen Stelle knickt, und sich darum runder um den Ambossfortsatz, den Hammergriff oder das längliche Aktor-Endstück legen lässt.

Damit ergibt sich zum einen eine Verringerung der Kraft, die der Chirurg zur Applikation aufbringen muss, und somit eine Verringerung des Risikos, dass der Ambossfortsatz oder der Hammergriff durch ein zu starkes Crimpen traumatisiert wird. Zum anderen werden aber auch eine Verringerung der oben diskutierten Auffederwirkung (mechanischer Memory-Effekt) und damit eine bessere Ankopplung der Gehörknöchelchenprothese aus akustischer Sicht erreicht.

Durch die länglichen Perforationen ist es dem Chirurgen möglich, mittels eines feinen Häkchens, die Schlinge quasi an den Incus anzumodellieren und so eine möglichst sichere Kontaktfläche zu erhalten. Diese Kontaktfläche führt später dazu, dass eine sichere akustische Signalübertragung stattfindet.

Sollte es wider Erwarten zu einer Revisionsoperation kommen und die Prothese aus dem Mittelohr entnommen werden müssen, sind die Perforationen wiederum von großem Vorteil, da mittels eines Häkchens die Prothese in den Perforationen gefasst werden und so sehr einfach vom Incus entfernt werden kann.
In der Praxis lassen sich die länglichen Perforationen in der Schlinge des ersten Ankoppelelements besonders präzise und unkompliziert durch Laserschneiden herstellen.

Eine besonders einfach herstellbare Klasse von Ausführungsformen der erfindungsgemäßen Gehörknöchelchenprothese ist dadurch gekennzeichnet, dass die länglichen Perforationen auf dem bandförmigen metallischen Material in dessen flachem Zustand gerade verlaufende Durchgangsschlitze umfassen. Nachdem die Schlinge am Incus anliegt, kann die den Incus überziehende Schleimhaut durch die Perforationen hindurch wachsen und so mittelfristig zu einer auch akustisch festen Verbindung führen. Ein weiterer Vorteil ist, dass die Prothese, sollte intra-operativ keine adäquate Ankopplung stattfinden, einfach mittels Kleber oder Zement durch die Perforationen hindurch festgeklebt werden kann

Bei einfachen Weiterbildungen dieser Ausführungsformen weisen die Durchgangsschlitze in ihrer Längsrichtung zwei parallele Seitenbegrenzungen sowie über den Längsverlauf gleichbleibende Schlitzbreiten auf. Die Schlitze sind also im Wesentlichen von länglicher Rechteckform.

Alternativ können sich aber auch bei anderen Weiterbildungen die Durchgangsschlitze in ihrer Längsrichtung verjüngen und in Verjüngungsrichtung kontinuierlich kleiner werdende Schlitzbreiten aufweisen.

Eine alternative Klasse von Ausführungsformen der Erfindung zeichnet sich dadurch aus, dass die länglichen Perforationen auf dem bandförmigen metallischen Material in dessen flachem Zustand schlangenlinienförmig oder zickzackförmig verlaufende Durchgangsschlitze umfassen.

Bei einer dritten Klasse von Ausführungsformen der erfindungsgemäßen Gehörknöchelchenprothese umfassen die länglichen Perforationen auf dem bandförmigen metallischen Material in dessen flachem Zustand mäanderförmig verlaufende Durchgangsschlitze. Auf diese Weise ist es unter anderem möglich, das Band auch in seiner Breite zu verjüngen, sollte dies aus patientenspezifischen anatomischen Gründen notwendig sein.

Besonders bevorzugt sind Ausführungsformen der erfindungsgemäßen Gehörknöchelchenprothese, die sich dadurch auszeichnen, dass die Schlinge mindestens zwei parallel verlaufende Reihen von jeweils in Richtung ihrer Längsachsen mit Abstand hintereinander angeordneten länglichen Perforationen aufweist. Durch diese Verbreiterung der Schlitzstruktur werden Druckspitzen, die auf das Gewebe und den Knochen wirken können, erheblich reduziert.

Bei vorteilhaften Weiterbildungen dieser Ausführungsformen weisen die länglichen Perforationen alle dieselbe Größe auf. Die gleiche Größe der Perforationen führt zu einer gleichmäßigeren Steifigkeit des ersten Ankoppelelements.

Zusätzlich oder alternativ können diese Ausführungsformen auch dahingehend weitergebildet werden, dass die länglichen Perforationen in einer Reihe auf Lücke in Längsrichtung versetzt zu den länglichen Perforationen einer benachbarten Reihe angeordnet sind. Damit wird erreicht, dass insbesondere bei der intra-operativen Bearbeitung des bandförmigen metallischen Materials keine Knickungen entstehen, da die Widerstandskraft konstant bleibt.

Vorteilhaft in der Handhabung und besonders kostengünstig in der Herstellung ist eine Klasse von Ausführungsformen der Erfindung, die dadurch gekennzeichnet sind, dass die Schlinge an einem freien Ende des bandförmigen metallischen Materials an der spaltartigen Öffnung einen Einführansatz zur Erleichterung des Aufbiegens oder Zudrückens der Schlinge mittels einer Crimpzange aufweist.

Diese Ausführungsformen lassen sich bei vorteilhaften Weiterbildungen noch dadurch verbessern, dass der Einführansatz der Schlinge gegenüber ihren gekrümmt verlaufenden Teilen, die im implantierten Zustand der Gehörknöchelchenprothese den Ambossfortsatz, den Hammergriff oder das Aktor-Endstück umgreifen, derart abgekröpft ist, dass er von der Längsachse des Ambossfortsatzes, des Hammergriffs oder des Aktor-Endstücks weg ragt. An dieser Stelle kann der Chirurg mit einem feinen Häkchen die Schlinge greifen und quasi um den Incus herumziehen, so dass die Schlinge möglichst direkt anliegt.

In der Praxis dürften bevorzugt Ausführungsformen der erfindungsgemäßen Gehörknöchelchenprothese eingesetzt werden, bei denen das zweite Ankoppelelement als Piston zum Eingriff ins Innenohr ausgestaltet ist.

Besonders bevorzugte Ausführungsformen der erfindungsgemäßen Gehörknöchelchenprothese sind dadurch gekennzeichnet, dass der Prothesenkörper mindestens ein Gelenk, insbesondere ein Kugelgelenk aufweist. Dies ist vorteilhaft im Hinblick auf eine besonders hohe postoperative Beweglichkeit der Prothese. Möglich sind auch Weiterbildungen, bei denen eine Vielzahl von aneinander angrenzenden weiteren Drehelementen vorgesehen ist, vorzugsweise eine Kugelgelenkkette. Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben.

Die erfindungsgemäße Gehörknöchelchenprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen die Gehörknöchelchenprothese oder Teile davon aus einem Material mit Formgedächtnis (= memory effect) und/oder superelastischen Eigenschaften, vorzugsweise aus Nitinol hergestellt sind, was per se beispielsweise aus der WO 02/069850 A1 oder der US 6,554,861 B2 bekannt ist.

Alternativ oder ergänzend können bei weiteren Ausführungsformen Teile der erfindungsgemäßen Gehörknöchelchenprothese aus einem Keramikmaterial hergestellt sein.

Möglich sind aber auch Ausführungsformen der Erfindung, bei denen die gesamte Prothese oder Teile davon aus biokompatiblen Kunststoffen, insbesondere Silikon, Polytetrafluorethylen (PTFE) oder Faserverbundwerkstoffen hergestellt sind. Mit diesen Materialien können postoperative Abstoßungsreaktionen in den meisten Fällen ebenfalls verhindert werden.

Besonders bevorzugt ist eine Ausführungsform der erfindungsgemäßen Gehörknöchelchenprothese, bei der die Massenverteilung der einzelnen Teile der Prothese in Abhängigkeit von einem gewünschten, vorgebbaren Frequenzgang der Schallleitung im Mittelohr berechnet ist. Damit lässt sich ohne großen zusätzlichen technischen Aufwand gewissermaßen ein Tuning der Schallfortpflanzungseigenschaften mittels einer individuellen ausgestalteten Gehörknöchelchenprothese erreichen. Die gezielte Frequenzanpassung zur verbesserten Schallleitung im Mittelohr ist beispielsweise in der EP 1 706 071 B1 oder in der US 7,871,439 B2 beschrieben.

Ein solches "mechanisches Tuning" kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist. Bei vorteilhaften Weiterbildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können jeweils einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:

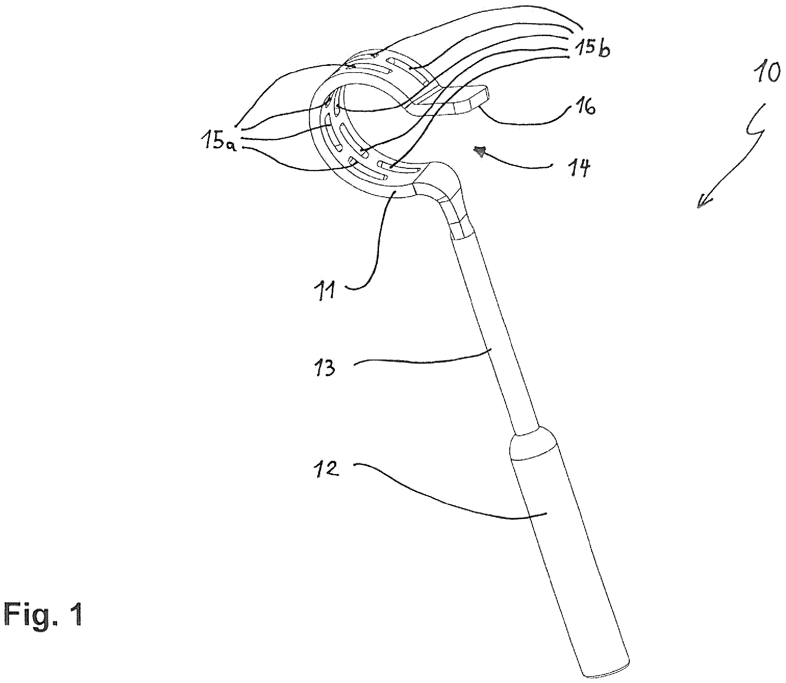

- Fig. 1: eine schematische räumliche Darstellung einer Ausführungsform der erfindungsgemäßen Gehörknöchelchenprothese aufrecht stehend schräg seitlich mit einem als Schlinge aus flachem, perforiertem Bandmaterial geformten ersten Ankoppelelement sowie einem als Piston ausgestaltetem zweiten Ankoppelelement zum Eingriff durch eine perforierte Steigbügel-Fußplatte hindurch ins Innenohr;
- Fig. 2: die Ausführungsform aus Fig. 1 liegend von der der spaltartigen Öffnung abgewandten Rückseite her gesehen; und
- Fig. 3: die Ausführungsform aus Fig. 1 in seitlicher Sicht mit Blickrichtung parallel zur Lage der Längsachse des Ambossfortsatzes, des Hammergriffs oder des Aktor-Endstücks im implantierten Zustand der Prothese.

Die in den Figuren der Zeichnung schematisch dargestellte Ausführungsform der erfindungsgemäßen **Gehörknöchelchenprothese 10** -die bei in der Zeichnung nicht dargestellten Ausführungsformen im Detail auch unterschiedlich gestaltet sein kannweist einen schaftförmigen länglichen, Schall übertragenden **Prothesenkörper 13** auf, welcher am einen Ende ein **erstes Ankoppelelement 11** trägt. Dieses ist zur mechanischen Verbindung mit dem Ambossfortsatz (="Incus"), mit dem Hammergriff ("Malleus") oder mit einem länglichen Aktor-Endstück eines aktiven Hörgeräts in Form einer das gewünschte Glied der Gehörknöchelchenkette oder das Aktor-Endstück umgebenden, durch eine **spaltartige Öffnung 14** nach außen hin teilweise offenen Schlinge aus bandförmigem metallischem Material gestaltet, welche postoperativ den Ambossfortsatz, den Hammergriff oder das Aktor-Endstück kraftschlüssig umgreift.

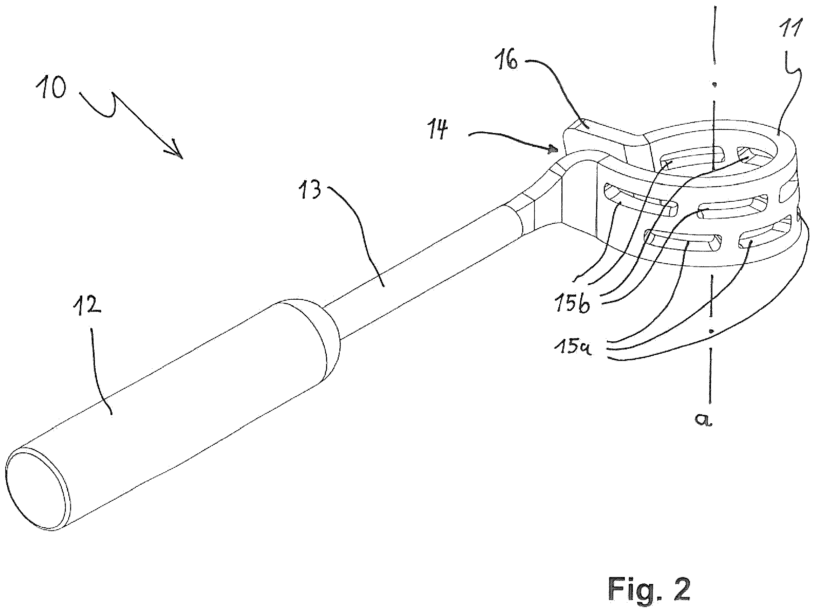

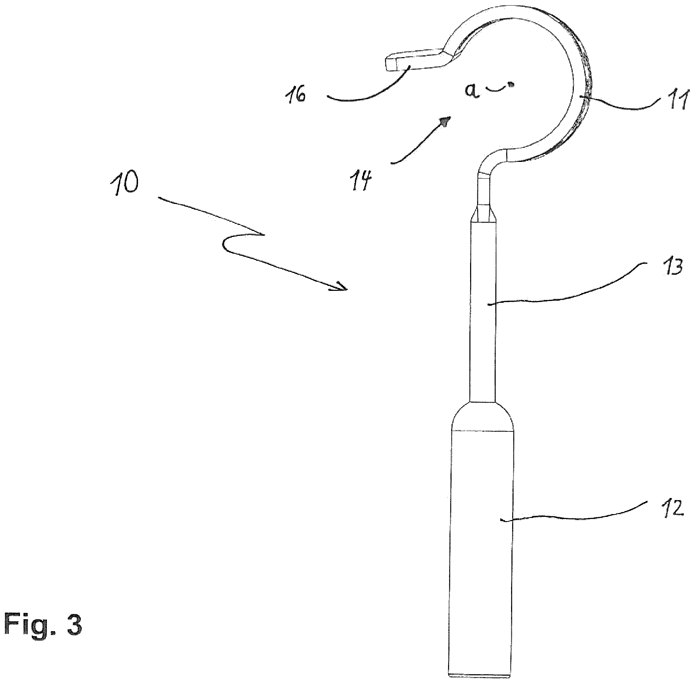

Das von der Schlinge zu umgreifende Glied der Gehörknöchelchenkette oder das Aktor-Endstück sind in der Zeichnung nicht gezeigt. Lediglich die **Längsachse a** des zu umgreifenden Ambossfortsatzes, des Hammergriffs oder des Aktor-Endstücks in ihrer Position relativ zur Gehörknöchelchenprothese 10 in deren im Mittelohr implantierten Zustand ist in Figur 2 als strichpunktierte Linie und in Figur 3 punktförmig als die Zeichenebene senkrecht durchdringende Achse bildlich dargestellt.

Am anderen Ende des Prothesenkörpers 13 sitzt ein **zweites Ankoppelelement 12,** welches in vielfältigen geometrischen Formen zur mechanischen Verbindung mit einem weiteren Glied oder Teilen eines Gliedes der Gehörknöchelchenkette oder -wie bei der in den Figuren der Zeichnung dargestellten Ausführungsform- als Piston zum direkten Eintauchen ins Innenohr gestaltet sein kann.

Erfindungsgemäß ist die Gehörknöchelchenprothese 10 dadurch gekennzeichnet, dass die Schlinge aus bandförmigem metallischem Material eine Vielzahl von **länglichen Perforationen 15a, 15b** aufweist, deren Längsachsen im implantierten Zustand der Gehörknöchelchenprothese 10 jeweils auf einer gekrümmten Bahn rechtwinklig oder schräg zu einer zur Längsachse a des von der Schlinge umgriffenen Ambossfortsatzes, Hammergriffs oder Aktor-Endstücks parallelen Achse verlaufen. Die länglichen Perforationen 15a, 15b in der Schlinge des ersten Ankoppelelements 11 können durch Laserschneiden hergestellt sein.

Bei der in den Figuren der Zeichnung dargestellten Ausführungsform der erfindungsgemäßen Gehörknöchelchenprothese 10 ist das erste Ankoppelelement 11 derart gestaltet, dass die Schlinge an einem freien Ende des bandförmigen metallischen Materials an der spaltartigen Öffnung 14 einen **Einführansatz 16** zur Erleichterung des Aufbiegens oder Zudrückens der Schlinge mittels einer Crimpzange aufweist. Der Einführansatz 16 der Schlinge ist gegenüber ihren gekrümmt verlaufenden Teilen, die im implantierten Zustand der erfindungsgemäßen Gehörknöchelchenprothese 10 den Ambossfortsatz, den Hammergriff oder das Aktor-Endstück umgreifen, derart abgekröpft, dass er von der Längsachse a des Ambossfortsatzes, des Hammergriffs oder des Aktor-Endstücks weg ragt.

Die in der Zeichnung gezeigte Ausführungsform zeichnet sich auch dadurch aus, dass die länglichen Perforationen 15a, 15b im bandförmigen metallischen Material in dessen flachem Zustand gerade verlaufende Durchgangsschlitze umfassen, die in ihrer Längsrichtung zwei parallele Seitenbegrenzungen sowie über den Längsverlauf gleichbleibende Schlitzbreiten aufweisen.

Alternativ dazu können -bei in der Zeichnung nicht dargestellten Ausführungsformen der Erfindung- die Durchgangsschlitze in ihrer Längsrichtung sich verjüngen und kontinuierlich kleiner werdende Schlitzbreiten aufweisen.

Bei anderen, ebenfalls nicht gezeigten Ausführungsformen können die länglichen Perforationen im bandförmigen metallischen Material in dessen flachem Zustand schlangenlinienförmig oder zickzackförmig verlaufende oder auch mäanderförmige Durchgangsschlitze umfassen.

Die in den Figuren der Zeichnung dargestellte Ausführungsform zeichnet sich weiterhin dadurch aus, dass die Schlinge zwei parallel verlaufende Reihen von jeweils in Richtung ihrer Längsachsen mit Abstand hintereinander angeordneten länglichen Perforationen 15a, 15b aufweist, die alle von derselben Größe sind, wobei die länglichen Perforationen 15a, 15b in einer Reihe auf Lücke in Längsrichtung versetzt zu den länglichen Perforationen 15b, 15a einer benachbarten Reihe angeordnet sind. Bei weiteren, hier nicht gezeigten Ausführungsformen der Erfindung können aber auch mehr als zwei Reihen von parallel verlaufenden länglichen Perforationen vorgesehen sein.

Der Prothesenkörper kann -bei in der Zeichnung nicht dargestellten Ausführungsformen der Erfindung- mindestens ein Gelenk, insbesondere ein Kugelgelenk aufweisen.

Die Massenverteilung der einzelnen Teile einer erfindungsgemäßen Gehörknöchelchenprothese kann in Abhängigkeit von einem gewünschten, vorgebbaren Frequenzgang der Schallleitung im Mittelohr derart berechnet sein, dass ein individuelles akustisches Tuning der Schallleitungs-Eigenschaften ermöglicht wird. Dies kann auch -bei in der Zeichnung wiederum nicht dargestellten Ausführungsformen der Erfindung- durch an die Gehörknöchelchenprothese anclipsbare "Trimm-Massen" erreicht werden.

Die erfindungsgemäße Gehörknöchelchenprothese 10 kann ganz oder teilweise aus einem Material mit Formgedächtnis (=memory effect) und/oder mit superelastischen Eigenschaften, insbesondere aus Nitinol hergestellt sein.

## Patentansprüche

1. Passive Gehörknöchelchenprothese (10), die zum Ersatz oder Überbrücken mindestens eines Glieds der menschlichen Gehörknöchelchenkette ausgebildet ist, mit einem Schall übertragenden Prothesenkörper (13), welcher an seinem einen Ende ein erstes Ankoppelelement (11) aufweist, das zur mechanischen Verbindung mit dem Ambossfortsatz (="Incus"), mit dem Hammergriff ("Malleus") oder mit einem länglichen Aktor-Endstück eines aktiven Hörgeräts in Form einer das Glied der Gehörknöchelchenkette oder das Aktor-Endstück umgebenden, durch eine spaltartige Öffnung (14) nach außen hin teilweise offenen Schlinge aus bandförmigem metallischem Material gestaltet ist, welche postoperativ den Ambossfortsatz, den Hammergriff oder das Aktor-Endstück kraftschlüssig umgreift und intraoperativ im Mittelohr zur Fixierung und dauerhaften Befestigung des ersten Ankoppelelements (11) am Ambossfortsatz, am Hammergriff oder am Aktor-Endstück mittels einer Crimpzange verformt werden kann, wobei der Prothesenkörper (13) an seinem anderen Ende ein zweites Ankoppelelement (12) zur mechanischen Verbindung mit einem weiteren Glied oder Teilen eines Gliedes der Gehörknöchelchenkette oder direkt mit dem Innenohr aufweist, und wobei die Schlinge aus bandförmigem metallischem Material eine Vielzahl von länglichen Perforationen (15a, 15b) aufweist, deren Längsachsen im implantierten Zustand der Gehörknöchelchenprothese (10) jeweils auf einer gekrümmten Bahn rechtwinklig oder schräg zu einer zur Längsachse (a) des Ambossfortsatzes, des Hammergriffs oder des Aktor-Endstücks parallelen Achse verlaufen,
**dadurch gekennzeichnet,**
**dass** die länglichen Perforationen (15a, 15b) jeweils in sich geschlossene Durchgangsschlitze sind,
**dass** die Schlinge mindestens zwei parallel verlaufende Reihen von jeweils mehreren in Richtung ihrer Längsachsen mit Abstand hintereinander angeordneten länglichen Perforationen (15a, 15b) aufweist,
und **dass** die länglichen Perforationen (15a, 15b) in einer Reihe auf Lücke in Längsrichtung versetzt zu den länglichen Perforationen (15b, 15a) einer benachbarten Reihe angeordnet sind.

2. Gehörknöchelchenprothese nach Anspruch 1, **dadurch gekennzeichnet, dass** die länglichen Perforationen (15a, 15b) im bandförmigen metallischen Material in dessen flachem Zustand gerade verlaufende Durchgangsschlitze umfassen.

3. Gehörknöchelchenprothese nach Anspruch 2, **dadurch gekennzeichnet, dass** die Durchgangsschlitze in ihrer Längsrichtung zwei parallele Seitenbegrenzungen sowie über den Längsverlauf gleichbleibende Schlitzbreiten aufweisen.

4. Gehörknöchelchenprothese nach Anspruch 2, **dadurch gekennzeichnet, dass** die Durchgangsschlitze in ihrer Längsrichtung sich verjüngen und kontinuierlich kleiner werdende Schlitzbreiten aufweisen.

5. Gehörknöchelchenprothese nach Anspruch 1, **dadurch gekennzeichnet, dass** die länglichen Perforationen im bandförmigen metallischen Material in dessen flachem Zustand schlangenlinienförmig oder zickzackförmig verlaufende Durchgangsschlitze umfassen.

6. Gehörknöchelchenprothese nach Anspruch 1, **dadurch gekennzeichnet, dass** die länglichen Perforationen im bandförmigen metallischen Material in dessen flachem Zustand mäanderförmig verlaufende Durchgangsschlitze umfassen.

7. Gehörknöchelchenprothese nach einem der vorhergehenden Ansprüche, **dadurch gekennzeichnet, dass** die länglichen Perforationen (15a, 15b) alle dieselbe Größe aufweisen.

8. Gehörknöchelchenprothese nach einem der vorhergehenden Ansprüche, **dadurch gekennzeichnet, dass** die Schlinge an einem freien Ende des bandförmigen metallischen Materials an der spaltartigen Öffnung (14) einen Einführansatz (16) zur Erleichterung des Aufbiegens oder Zudrückens der Schlinge mittels einer Crimpzange aufweist.

9. Gehörknöchelchenprothese nach Anspruch 8 **dadurch gekennzeichnet, dass** der Einführansatz (16) der Schlinge gegenüber ihren gekrümmt verlaufenden Teilen, die im implantierten Zustand der Gehörknöchelchenprothese (10) den Ambossfortsatz, den Hammergriff oder das Aktor-Endstück umgreifen, derart abgekröpft ist, dass er von der Längsachse (a) des Ambossfortsatzes, des Hammergriffs oder des Aktor-Endstücks weg ragt.

10. Gehörknöchelchenprothese nach einem der vorhergehenden Ansprüche, **dadurch gekennzeichnet, dass** die länglichen Perforationen (15a, 15b) in der Schlinge durch Laserschneiden hergestellt sind.

11. Gehörknöchelchenprothese nach einem der vorhergehenden Ansprüche, **dadurch gekennzeichnet, dass** das zweite Ankoppelelement (12) als Piston zum Eingriff ins Innenohr ausgestaltet ist.

12. Gehörknöchelchenprothese nach einem der vorhergehenden Ansprüche, **dadurch gekennzeichnet, dass** die Gehörknöchelchenprothese (10) ganz oder teilweise aus einem Material mit Formgedächtnis (=memory effect) und/oder mit superelastischen Eigenschaften, insbesondere aus Nitinol hergestellt ist.

13. Gehörknöchelchenprothese nach einem der vorhergehenden Ansprüche, **dadurch gekennzeichnet, dass** der Prothesenkörper (13) mindestens ein Gelenk, insbesondere ein Kugelgelenk aufweist.

## Claims

1. Passive auditory ossicle prosthesis (10) for replacing or bridging at least one member of the human ossicular chain, comprising a sound-transmitting prosthesis body (13) having at one end a first coupling element (11) which is designed for mechanical connection to the incus, to the malleus or to an elongated actuator end-piece of an active hearing aid, said first coupling element (11) being in the form of a loop made of strip-shaped metal material which surrounds the member of the ossicular chain or the actuator end-piece, which loop is partially open to the outside through a gap-like opening (14) and postoperatively engages non-positively around the incus, the malleus or the actuator end-piece, and intraoperativly can be deformed by means of a crimping tool in the middle ear to fix and permanently fasten the first coupling element (11) to the incus, the malleus or the actuator end-piece, wherein the prosthesis body (13) has at its other end a second coupling element (12) for mechanical connection to a further member or parts of a member of the ossicular chain or directly to the inner ear, and wherein the loop of strip-shaped metal material has a plurality of elongated perforations (15a, 15b) the longitudinal axes of which, in the implanted state of the auditory ossicle prosthesis (10), each extend on a curved path at right angles or obliquely with respect to an axis parallel to the longitudinal axis (a) of the incus, the malleus or the actuator end-piece,
**characterised in that**
the elongated perforations (15a, 15b) are each through-slits closed within themselves,
**in that** the loop has at least two parallel rows made up of a plurality of elongated perforations (15a, 15b) spaced one behind the other in the direction of their longitudinal axes,
and **in that** the elongated perforations (15a, 15b) of one row are staggered with respect to the elongated perforations (15b, 15a) of a neighbouring row.

2. Auditory ossicle prosthesis according to Claim 1, **characterised in that** the elongated perforations (15a, 15b) in the strip-shaped metal material include rectilinear through-slits in the flat state of the strip-shaped metal material.

3. Auditory ossicle prosthesis according to Claim 2, **characterised in that** the through-slits have two parallel lateral delimitations in their longitudinal direction and have constant slit widths over their longitudinal extent.

4. Auditory ossicle prosthesis according to Claim 2, **characterised in that** the through-slits taper in the longitudinal direction and have continuously diminishing widths.

5. Auditory ossicle prosthesis according to Claim 1, **characterised in that** the elongated perforations include sinuous or zigzag-shaped through-slits in the flat state of the strip-shaped metal material.

6. Auditory ossicle prosthesis according to Claim 1, **characterised in that** the elongated perforations include meandering through-slits in the flat state of the strip-shaped metal material.

7. Auditory ossicle prosthesis according to any one of the preceding claims, **characterised in that** the elongated perforations (15a, 15b) are all of the same size.

8. Auditory ossicle prosthesis according to any one of the preceding claims, **characterised in that**, at a free end of the strip-shaped metal material, the loop has at its gap-like opening (14) an insertion shoulder (16) to facilitate bending the loop open or pressing it closed by means of a crimping tool.

9. Auditory ossicle prosthesis according to Claim 8, **characterised in that** the insertion shoulder (16) of the loop is bent away in relation to the curved parts of the loop which engage around the incus, the malleus or the actuator end-piece in the implanted state of the auditory ossicle prosthesis (10), in such a way that it projects away from the longitudinal axis (a) of the incus, the malleus or the actuator end-piece.

10. Auditory ossicle prosthesis according to any one of the preceding claims, **characterised in that** the elongated perforations (15a, 15b) are produced in the loop by laser cutting.

11. Auditory ossicle prosthesis according to any one of the preceding claims, **characterised in that** the second coupling element (12) is configured as a piston for engaging in the inner ear.

12. Auditory ossicle prosthesis according to any one of the preceding claims, **characterised in that** the auditory ossicle prosthesis (10) is produced wholly or partially from a material having memory effect and/or having superelastic properties, in particular from nitinol.

13. Auditory ossicle prosthesis according to any one of the preceding claims, **characterised in that** the prosthesis body (13) includes at least one joint, in particular a ball-and-socket joint.

## Revendications

1. Prothèse passive d'osselet auditif (10), qui est réalisée pour le remplacement ou pour le pontage d'au moins un membre de la chaîne d'osselets auditifs humains, comprenant un corps de prothèse (13) qui transmet les sons et qui comprend à l'une de ses extrémités un premier élément de couplage (11), lequel est conçu, pour la liaison mécanique avec le prolongement d'enclume (= "Incus"), avec le marteau ("Malleus") ou avec une pièce terminale allongée formant acteur d'un appareil auditif actif, sous la forme d'une boucle partiellement ouverte vers l'extérieur à travers une ouverture (14) en forme de fente, ladite boucle entourant le membre de la chaîne d'osselets auditifs ou la pièce terminale formant acteur et étant réalisée en un matériau métallique en forme de bande, ladite boucle entourant en situation postopératoire l'enclume, le marteau ou la pièce terminale formant acteur, et pouvant être déformée par voie intraopératoire dans l'oreille moyenne pour la fixation et l'immobilisation durable du premier élément de couplage (11) sur le prolongement de l'enclume, sur le marteau ou sur la pièce terminale formant acteur au moyen d'une pince de sertissage, dans laquelle le corps de prothèse (13) comprend à son autre extrémité un second élément de couplage (12) pour la liaison mécanique avec un autre membre ou avec des parties d'un membre de la chaîne d'osselets auditifs ou encore directement avec l'oreille interne, et dans laquelle la boucle en matériau métallique en forme de bande comporte une pluralité de perforations oblongues (15a, 15b), dont les axes longitudinaux s'étendent, dans l'état implanté de la prothèse d'osselet auditif (10), respectivement sur une voie incurvée, à angle droit ou en oblique par rapport à un axe parallèle à l'axe longitudinal (a) du prolongement de l'enclume, du marteau ou de la pièce terminale formant acteur, **caractérisée en ce que**
les perforations oblongues (15a, 15b) sont respectivement des fentes traversantes fermées sur elles-mêmes,
**en ce que** la boucle comprend au moins deux rangées parallèles formées respectivement de plusieurs perforations oblongues (15a, 15b) agencées à distance les unes derrière les autres en direction de leurs axes longitudinaux,
et **en ce que** les perforations oblongues (15a, 15b) sont agencées dans une rangée en quinconce, décalées en direction longitudinale par rapport aux perforations oblongues (15b, 15a) d'une rangée voisine.

2. Prothèse d'osselet auditif selon la revendication 1, **caractérisée en ce que** les perforations oblongues (15a, 15b) en matériau métallique en forme de bande incluent, dans l'état à plat, des fentes traversantes qui s'étendent en ligne droite.

3. Prothèse d'osselet auditif selon la revendication 2, **caractérisée en ce que** les fentes traversantes présentent, dans leur direction longitudinale, deux délimitations latérales parallèles, ainsi que des largeurs de fentes qui restent constantes sur le tracé en longueur.

4. Prothèse d'osselet auditif selon la revendication 2, **caractérisée en ce que** les fentes traversantes présentent, dans la direction longitudinale, des largeurs de fente qui vont en se rétrécissant et qui deviennent continuellement plus petites.

5. Prothèse d'osselet auditif selon la revendication 1, **caractérisée en ce que** les perforations oblongues dans le matériau métallique en forme de bande incluent, dans son état à plat, des fentes traversantes en forme de ligne en serpentin ou s'étendant en forme de zigzag.

6. Prothèse d'osselet auditif selon la revendication 1, **caractérisée en ce que** les perforations oblongues dans le matériau métallique en forme de bande incluent, dans son état à plat, des fentes traversantes qui s'étendent en formant des méandres.

7. Prothèse d'osselet auditif selon l'une des revendications précédentes, **caractérisée en ce que** les perforations oblongues (15a, 15b) ont toute la même taille.

8. Prothèse d'osselet auditif selon l'une des revendications précédentes, **caractérisée en ce que** la boucle à une extrémité libre du matériau métallique en forme de bande présente, au niveau de l'ouverture en forme de fente (14), une amorce d'introduction (16) pour faciliter la flexion ou l'écrasement de la boucle au moyen d'une pince de sertissage.

9. Prothèse d'osselet auditif selon la revendication 8, **caractérisée en ce que** l'amorce d'introduction (16) de la boucle est coudée, par rapport à ses parties incurvées, qui entourent dans l'état implanté de la prothèse d'osselet auditif (10) le prolongement d'enclume, le marteau ou la pièce terminale formant acteur, de telle façon qu'elle est dirigée en éloignement de l'axe longitudinal (a) du prolongement d'enclume, du marteau ou de la pièce terminale formant acteur.

10. Prothèse d'osselet auditif selon l'une des revendications précédentes, **caractérisée en ce que** les perforations oblongues (15a, 15b) dans la boucle sont produites par découpe au laser.

11. Prothèse d'osselet auditif selon l'une des revendications précédentes, **caractérisée en ce que** le second élément de couplage (12) est conçu sous la forme d'un piston pour s'engager dans l'oreille interne.

12. Prothèse d'osselet auditif selon l'une des revendications précédentes, **caractérisée en ce que** la prothèse d'osselet auditif (10) est fabriquée en totalité ou en partie en un matériau à mémoire de forme (= memory effect) et/ou avec des propriétés superélastiques, en particulier en nitinol.

13. Prothèse d'osselet auditif selon l'une des revendications précédentes, **caractérisée en ce que** le corps de prothèse (13) comprend au moins une articulation, en particulier une articulation à rotule.
